# EUROPEAN PATENT APPLICATION

(11) **EP 2 037 275 A1**
(43) Date of publication of application: **18.03.2009**
(21) Application number: 07116150.9
(22) Date of filing: 11.09.2007
(51) Int. Cl.: G01N 33/68

(54) **Differentiation of different causes of right heart failure**

(71) Applicant: F. Hoffmann-La Roche AG, 4070 Basel (CH); Roche Diagnostics GmbH, 68305 Mannheim (DE)
(72) Inventor: The designation of the inventor has not yet been filed
(74) Representative: Dick, Alexander

(57) **Abstract**

The present invention relates to the field of diagnostic means and methods. More specifically, the present invention relates to a method of differentiating between pulmonary embolism and pulmonary hypertension as the cause of right heart failure in a subject comprising determining the amounts of a natriuretic peptide, a cardiac troponin, GDF-15 and Endoglin in a sample of a subject suffering from right heart failure and comparing the said amounts with reference amounts, whereby it is differentiated between pulmonary embolism and pulmonary hypertension as the cause of the right heart failure. Furthermore, the present invention relates to methods of determining whether a subject suffering from right heart failure is susceptible to a therapy for pulmonary hypertension or pulmonary embolism as well as to a diagnostic device and a diagnostic kit adapted for carrying out the method of the present invention.

## Description

The present invention relates to the field of diagnostic means and methods. More specifically, the present invention relates to a method of differentiating between pulmonary embolism and pulmonary hypertension as the cause of right heart failure in a subject comprising determining the amounts of a natriuretic peptide, a cardiac troponin, GDF-15 and Endoglin in a sample of a subject suffering from right heart failure and comparing the said amounts with reference amounts, whereby it is differentiated between pulmonary embolism and pulmonary hypertension as the cause of the right heart failure. Furthermore, the present invention relates to methods of determining whether a subject suffering from right heart failure is susceptible to a therapy for pulmonary hypertension or pulmonary embolism as well as to a diagnostic device and a diagnostic kit adapted for carrying out the method of the present invention.

Right heart failure, also known as *cor pulmonale*, is a medical condition accompanied by a change in structure and function of the right ventricle of the heart as a result of, e.g., a respiratory disorder. If left untreated, right heart failure is a life threatening disease.

Right ventricular hypertrophy is one predominant change in chronic right heart failure. However, in acute right heart failure dilation may occur as well. Both changes are caused by the increased right ventricular blood pressure. Dilation is essentially a stretching of the ventricle, the immediate result of increasing the pressure in an elastic container. Ventricular hypertrophy is an adaptive response to a long-term increase in pressure as observed in chronic right heart failure patients requiring heart muscle growth. The additional heart muscle cells are necessary to allow for the increased contractile force which is required for moving the blood against the increased pulmonary resistance.

The pivotal causes of right heart failure are disorders of the pulmonary circulation. Specifically, the pulmonary circulation may be affected by a pulmonary embolism or by pulmonary hypertension. It is to be understood that these causes, although both resulting in right heart failure, require different medical care and treatment regimens.

Pulmonary embolism is usually accompanied by the characteristic clinical symptoms acute shortness of breath, collapse-like conditions and chest pain. Pulmonary embolism is caused by thrombosis which often occurs in femoral veins. Moreover, the thrombosis may be accompanied by further diseases such as genetically caused defects in the blood coagulation cascade or cancer diseases. As a consequence of thrombosis, a floating thrombus may enter and occlude the lung artery. The size of the embolus determines the position of the arterial occlusion.

Pulmonary hypertension or, more specifically, pulmonary arterial hypertension is defined as a sustained elevation of pulmonary arterial blood pressure of at leas 25 mmHg at rest and more than 30 mmHg with exercise. Different causes of pulmonary hypertension have been reported. A classification of the disease can be found in Farber et al. (Farber 2004, N Engl J Med 351: 1655-1665).

A differentiation of the cause of right heart failure is required, whatsoever, in order to select an efficient therapeutic measure for a patient. However, suitable means and methods for diagnostically discriminating between the two causes are not yet available. Risk stratification methods based on the combination of echocardiography and the N-terminal pro-Brain natriuretic peptide or the cardiac troponins Troponin I and T as biomarkers have been reported (Binder 2005, Circulation 112: 1573-1579; Konstantinides 2002, Circulation 106:1263-1268). These methods, however, require a cumbersome echocardiographic monitoring of the patients and do not aim to differentiate between the causes of the right heart failure in a first instance.

Thus, the technical problem underlying the present invention may be seen as the provision of means and methods for complying with the aforementioned needs. The technical problem is solved by the embodiments characterized in the claims and herein below.

Accordingly, the present invention relates to a method of differentiating between pulmonary embolism and pulmonary hypertension as the cause of right heart failure in a subject comprising:
a) determining the amounts of a natriuretic peptide, a cardiac troponin, GDF-15 and Endoglin in a sample of a subject suffering from right heart failure; and
b) comparing the amounts determined in step a) with reference amounts, whereby its differentiated between pulmonary embolism and pulmonary hypertension as the cause of the right heart failure.

The method of the present invention is, preferably, an in vitro method. Moreover, it is to be understood that the method may comprise additional steps such as sample pre-treatment steps. The method is also preferably assisted by automation. Step a) may be assisted by robotic and analyzing devices while step b) may be assisted by a computer comprising a computer program code which implements an algorithm suitable for the comparison of the aforementioned amounts.

The term "differentiating" as used herein means distinguishing between pulmonary hypertension and pulmonary embolism as the cause of an apparent right heart failure in a subject. The term as used herein, preferably, includes differentially diagnosing each condition. Diagnosing as used herein refers to assessing the probability according to which a subject suffers from the diseases referred to in this specification. As will be understood by those skilled in the art, such an assessment is usually not intended to be correct for 100% of the subjects to be diagnosed. The term, however, requires that a statistically significant portion of the subjects can be diagnosed to suffer from the said disease (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

Diagnosing according to the present invention also includes monitoring, confirmation, subclassification and prediction of the relevant disease, symptoms or risks therefor. Monitoring relates to keeping track of an already diagnosed disease, or complication, e.g. to analyzing the progression of the disease or the influence of a particular treatment on the progression of disease or complication. Confirmation relates to the strengthening or substantiating a diagnosis already performed using other indicators or markers. Subclassification relates to further defining a diagnosis according to different subclasses of the diagnosed disease, e.g. defining according to mild and severe forms of the disease. Prediction relates to prognosing a disease or complication before other symptoms or markers have become evident or have become significantly altered.

The term "right heart failure" as used herein relates to an impaired function of the right heart system. In its acute stage, right heart failure is accompanied by a dilation of the right ventricle as immediate response to an increased intraventricular blood pressure caused by an elevated pulmonary resistance. In its chronic stage, right heart failure is also accompanied by morphological changes including a hypertrophic myocardium within the right ventricle. However, in both stages the pulmonary blood flow becomes impaired having potentially life threatening consequences. Furthermore, a right ventricular volume overload which occurs during right heart failure may be accompanied by a poor support of the left heart as well.

The term "pulmonary embolism" as used herein refers to a disease or condition accompanied by acute shortness of breath, collapse-like conditions and/or chest pain. The pulmonary embolism as referred to in accordance with the present invention is, preferably, the result of an occlusion or stenosis of pulmonary blood vessels. Pulmonary embolism encompasses singular as well as multiple occlusion or stenosis events which result in socalled singular and multiple pulmonary embolisms, respectively.

In accordance with the present invention, the term "pulmonary hypertension" refers to a medical condition characterized by a sustained elevation of pulmonary arterial blood pressure of at least 25 mmHg at rest and more than 30 mmHg with exercise. The main vascular changes observed in subjects exhibiting pulmonary hypertension are increased vasoconstriction, increased smooth-muscle cell and endothelial cell proliferation as well as thrombosis. Pulmonary hypertension can be classified according to the World Health Organization (WHO) into five groups: I) pulmonary arterial hypertension, II) pulmonary venous hypertension, III) pulmonary hypertension associated with hypoxemia, IV) pulmonary hypertension due to chronic thrombotic disease, embolic disease or both, V) miscellaneous including sarcoidosis, pulmonary Langerhans'-cell histiocytosis, lymphangiomatosis, compression of pulmonary vessels (see Farber 2004, N Engl J Med 351: 1655-1665).

The term "subject" as used herein relates to animals, preferably mammals, and, more preferably, humans. However, it is envisaged by the present invention that the subject shall, preferably, exhibit the aforementioned apparent clinical symptoms of a right heart failure.

Determining the amount of a polypeptide referred to herein (i.e. a natriuretic peptide, a cardiac troponin, GDF-15 or Endoglin) according to the present invention relates to measuring the amount or concentration, preferably semi-quantitatively or quantitatively. Measuring can be done directly or indirectly. Direct measuring relates to measuring the amount or concentration of the polypeptide based on a signal which is obtained from the polypeptide itself and the intensity of which directly correlates with the number of molecules of the peptide present in the sample. Such a signal - sometimes referred to herein as intensity signal - may be obtained, e.g., by measuring an intensity value of a specific physical or chemical property of the polypeptide. Indirect measuring includes measuring of a signal obtained from a secondary component (i.e. a component not being the polypeptide itself) or a biological read out system, e.g., measurable cellular responses, ligands, labels, or enzymatic reaction products.

In accordance with the present invention, determining the amount of the polypeptide can be achieved by all known means for determining the amount of a peptide in a sample. Said means comprise immunoassay devices and methods which may utilize labled molecules in various sandwich, competition, or other assay formats. Said assays will develop a signal which is indicative for the presence or absence of the polypeptide. Moreover, the signal strength can, preferably, be correlated directly or indirectly (e.g. reverse- proportional) to the amount of polypeptide present in a sample. Further suitable methods comprise measuring a physical or chemical property specific for the polypeptide such as its precise molecular mass or NMR spectrum. Said methods comprise, preferably, biosensors, optical devices coupled to immunoassays, biochips, analytical devices such as mass-spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a polypeptide comprises the steps of (a) contacting a cell capable of eliciting a cellular response the intensity of which is indicative of the amount of the polypeptide with the polypeptide for an adequate period of time, (b) measuring the cellular response.

For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the polypeptide.

Also preferably, determining the amount of the polypeptide comprises the step of measuring a specific intensity signal obtainable from the polypeptide or a pulmonary surfactant protein in the sample.

As described above, such a signal may be the signal intensity observed at an m/z variable specific for the polypeptide observed in mass spectra or a NMR spectrum specific for the polypeptide.

Further, determining the amount of a polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the polypeptides described herein. Preferred ligands include antibodies, nucleic acids, peptides or polypeptides such as receptors for the polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the polypeptide. Specific binding according to the present invention means that the ligand or agent should not bind substantially to ("cross-react" with) another peptide, polypeptide or substance present in the sample to be analyzed. Preferably, the specifically bound polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative.

Suitable methods are described in the following. First, binding of a ligand may be measured directly, e.g. by NMR, mass spectrometry or surface plasmon resonance. Second, if the ligand also serves as a substrate of an enzymatic activity of the polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the ligand/polypeptide complex or the ligand which was bound by the polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

Furthermore preferably, determining the amount of a polypeptide comprises (a) contacting a solid support comprising a ligand for the polypeptide as specified above with a sample comprising the polypeptide and (b) measuring the amount of the polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of the polypeptides referred to herein, the relative amount or concentration of the polypeptides referred to herein as well as any value or parameter which correlates thereto. Such values or parameters comprise intensity signal values from all specific physical or chemical properties obtained from the polypeptide referred to herein by direct measurements, e.g., intensity values in mass spectra or NMR spectra. Moreover, encompassed are all values or parameters which are obtained by indirect measurements specified elsewhere in this description, e.g., expression levels determined from biological read out systems in response to the polypeptides referred to herein or intensity signals obtained from specifically bound ligands. It is to be understood that values correlating to the aforementioned amounts or parameters can also be obtained by all standard mathematical operations.

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493. The term "cardiac Troponin" encompasses also variants of the aforementioned specific Troponins, i.e., preferably, of Tropoinin T or Troponin I. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Troponin. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Such fragments may be, e.g., degradation products of the Troponins. Further included are variants which differ due to posttranslational modifications such as phosphorylation or myristylation.

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated gene-1, and prostate-derived factor (Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a -28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as glycosylation or myristylation.

The term "Endoglin" as used herein refers to a polypeptide having a molecular weight of 180 kDa non-reduced, 95 kDa after reduction and 66 kDa in its reduced and N-deglycosylated form. The polypeptide is capable of forming dimmers and bins to TGF-β and TGF-β receptors (see below). Endoglin may be phosphorylated. Preferably, Endoglin refers to human Endoglin. More preferably, human Endoglin has an amino acid sequence as shown in Genebank accession number AAC63386.1, GI: 3201489. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Endoglin. Variants may be allelic variants, splice varaiants or any other species specific homologs, paralogs, or orthologs. Moreover, the variants referred to herein include fragments of the specific Endoglin or the aforementioned types of variants as long as these fragments have the essential immunological and biological properties as referred to above. Such fragments may be, e.g., degradation products of Endoglin. Further included are variants which differ due to posttranslational modifications such as glycosylation, phosphorylation or myristylation.

The term "sample" refers to a sample of a body fluid, to a sample of separated cells or to a sample from a tissue or an organ. Samples of body fluids can be obtained by well known techniques and include, preferably, samples of blood, plasma, serum or urine. Tissue or organ samples may be obtained from any tissue or organ by, e.g., biopsy. Separated cells may be obtained from the body fluids or the tissues or organs by separating techniques such as centrifugation or cell sorting.

Comparing as used herein encompasses comparing the amount of the polypeptides referred to herein which are comprised by the sample to be analyzed with an amount of the said polypeptides in a suitable reference sample as specified below in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison by means of an expert system. Accordingly, a differential diagnosis for the diseases referred to herein may be automatically provided in a suitable output format.

The term "reference amount" as used herein refers to an amount which allows assessing which of the aforementioned diseases or disorders is the cause of right heart failure by a comparison as referred to above. Accordingly, the reference may either be derived from a subject known to suffer from right heart failure caused by a pulmonary embolism or known to suffer from right heart failure caused by pulmonary hypertension. It is to be understood that if a reference sample from a subject is used which suffers from right heart failure known to be caused by a pulmonary embolism, an amount of the polypeptides in a sample of a test subject being essentially identical to the amounts determined in the said reference sample (i.e. the reference amounts) shall be indicative for a pulmonary embolism as the cause of the right heart failure. Likewise, if a reference sample from a subject is used which suffers from right heart failure known to be caused by a pulmonary hypertension, an amount of the polypeptides in a sample of a test subject being essentially identical to the amounts determined in the said reference sample (i.e. the reference amounts) shall be indicative for a pulmonary hypertension as the cause of the right heart failure. The reference amount applicable for an individual subject may vary depending on various physiological parameters such as age, gender, or subpopulation. Thus, a suitable reference amount may be determined by the method of the present invention from a reference sample to be analyzed together, i.e. simultaneously or subsequently, with the test sample. Moreover, a threshold amount can be preferably used as a reference amount. An amount of the polypeptides which is above the threshold amount or equal or below the threshold amount will be indicative for either pulmonary hypertension or pulmonary embolism as the cause of the right heart failure. It has been found that reference amounts defining thresholds are (i) for the natriuretic peptide: 2500 pg/ml, (ii) for the cardiac troponin: 50 pg/ml, (iii) for GDF-15: 2000 pg/ml and (iv) for Endoglin: 5 ng/ml. It is to be understood that the aforementioned amounts may vary due to statistics and errors of measurement. Amounts of the natriuretic peptide, the cardiac troponin and GDF15 larger than or equal to the aforementioned reference amounts in combination with an amount of Endoglin less than the reference amount are indicative for a pulmonary embolism as the cause of the said right heart failure whereas amounts of the natriuretic peptide, the cardiac troponin and GDF15 less than the reference amounts in combination with an amount of Endoglin equal or larger than the reference amount are indicative for a pulmonary hypertension as the cause of the said right heart failure.

Advantageously, it has been found in the studies underlying this invention that a combination of the aforementioned polypeptides as biomarkers is suitable of discriminating between the different causes of right heart failure. It is to be understood that based on the cause of the right heart failure an appropriate therapy can be chosen. Thanks to the present invention, subjects and, in particular, emergency patients can be more readily and reliably diagnosed and subsequently correctly treated according to the result of the said differential diagnosis.

The explanations and definitions of the terms made above and herein below apply accordingly for all embodiments characterized in this specification and the claims.

The following embodiments are particularly preferred embodiments of the method of the present invention.

In a preferred embodiment of the method of the present invention, the reference amounts are (i) for the natriuretic peptide: 2500 pg/ml, (ii) for the cardiac troponin: 50 pg/ml, (iii) for GDF-15: 2000 pg/ml and (iv) for Endoglin: 5 ng/ml.

More preferably, amounts of the natriuretic peptide, the cardiac troponin and GDF15 larger than or equal to the said reference amounts and an amount of Endoglin less than the reference amount are indicative for a pulmonary embolism as the cause of the said right heart failure whereas amounts of the natriuretic peptide, the cardiac troponin and GDF15 less than the said reference amounts and an amount of Endoglin larger than or equal to the reference amount are indicative for a pulmonary hypertension as the cause of the said right heart failure.

It follows from the above that the present invention also encompasses a method of determining whether a subject suffering from right heart failure is susceptible to a therapy for pulmonary hypertension comprising the steps of the above method and the further step of identifying a subject susceptible to a therapy for pulmonary hypertension if pulmonary hypertension was determined as the cause of the said right hart failure.

The term "susceptible" as used herein means that a statistically significant portion of subjects identified by the method as being susceptible respond to the envisaged therapy by at least an amelioration of the right heart failure and the underlying disease being its cause. A amelioration can be identified by a reduction of the symptoms associated with either the right heart failure or its underlying cause.

Preferred therapies for pulmonary hypertension are selected from the group consisting of: vasodilator therapies using oxygen, calcium channel blockers, endothelin receptor blockers, natriuretic peptides or calcitonin gene-related peptides; antiinfammatory therapies using prostacyclic analogues, nitric oxide donors, endothelin receptor antagonists, statins, 5-lipoxygenase inhibitors or monocyte-macrophage chemoattractant protein-1; remodelling therapies using nitric oxide donors or endothelin receptor antagonists; inhalation therapies using oxygen, prostacyclin analogues, nitric oxide donors or ethyl nitrite; anticoagulant therapies; and antiplatelet therapies using prostacyclin analogues, nitic oxide donors, L-arginine, phosphodiesterase inhibitors or prostacyclin synthase. All therapies are well known and currently practiced.

Further, the present invention relates to a method of determining whether a subject suffering from right heart failure is susceptible to a therapy for pulmonary embolism comprising the steps of the above method and the further step of identifying a subject susceptible to a therapy for pulmonary embolism if pulmonary embolism was determined as the cause of the said right hart failure.

Preferably, said therapy for pulmonary embolism is a drug-based thrombolytic therapy or a surgery-based therapy. Thrombolytic therapies encompass, preferably, administration of thrombolytic drugs such as recombinant tissue plasminogen activator (rtPA). In this context, surgery-based therapies aim to remove the occlusion or stenosis of the blood vessel resulting in the pulmonary embolism.

The present invention also relates to a device for differentiating between pulmonary embolism and pulmonary hypertension as the cause of right heart failure in a subject comprising:
a) means for determining the amounts of a natriuretic peptide, a cardiac troponin, GDF-15 and Endoglin in a sample of a subject suffering from right heart failure; and
b) means for comparing the amounts determined in step a) with reference amounts, whereby its differentiated between pulmonary embolism and pulmonary hypertension as the cause of the right heart failure.

The term "device" as used herein relates to a system of means comprising at least the aforementioned means operatively linked to each other as to allow the prediction. Preferred means for determining the amount of the said polypeptides and means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to diagnose or distinguish between the diseases referred to herein. Preferably, the means are comprised by a single device in such a case. Said device may accordingly include an analyzing unit for the measurement of the amount of the peptides in a sample and a computer unit for processing the resulting data for the differential diagnosis. Alternatively, where means such as test stripes are used for determining the amount of the polypeptides, the means for diagnosing may comprise control stripes or tables allocating the determined amount to an amount known to be accompanied with pulmonary embolism or pulmonary hypertension as the cause of right heart failure. The test stripes are, preferably, coupled to a ligand which specifically binds to the polypeptides as defined elsewhere in this specification. The strip or device, preferably, comprises means for detection of the binding of said peptides to the said ligand. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonace devices, NMR spectrometers, mass- spectrometers etc.) or evaluation units/devices referred to above in accordance with the method of the invention.

Finally, the present invention relates to a kit adapted for carrying out the method of the present invention comprising:
a) means for determining the amounts of a natriuretic peptide, a cardiac troponin, GDF-15 and Endoglin in a sample of a subject suffering from right heart failure; and
b) means for comparing the amounts determined in step a) with reference amounts, whereby its differentiated between pulmonary embolism and pulmonary hypertension as the cause of the right heart failure.

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a polypeptide referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined polypeptides to the kind of diagnosis. Details are to be found elsewhere in this specification. Additionally, such user's manual may provide instructions about correctly using the components of the kit for determining the amount(s) of the respective biomarker. A users manual may be provided in paper or electronic form, e.g., stored on CD or CD ROM. The present invention also relates to the use of said kit in any of the methods according to the present invention.

All references cited in this specification are herewith incorporated by reference with respect to their entire disclosure content and the disclosure content specifically mentioned in this specification.

The following Example merely illustrates the invention. It shall, whatsoever, not be construed as a limitation of the scope of the invention.

**Example:** Determination of the biomarkers NT-proBNP, sensitive Troponin T, GDF-15 and Endoglin in patients exhibiting right heart failure

The plasma levels of NT-proBNP, sensitive Troponin T, GDF-15 and Endoglin were measured in patients exhibiting right heart failure either caused by pulmonary hypertension (number of patients n=55) or pulmonary embolism (number of patients n=17). Right heart failure and pulmonary embolism and pulmonary hypertension, respectively, were confirmed by echocardiography in combination with spiral CT-scans and pulmonary angiography. For determination of NT-proBNP, the Elecsys^{™} test (Roche Diagnostics, Germany) was used. The other biomarkers were determined by the Elecsys Troponin T 3rd generation Assay (Roche Diagnostics, Germany) and the Quantikine Human Endoglin/CD105 Immunoassay (cat. no.: DNDG00; R&D Systems, Inc., USA). GDF-15 was determined as described in Kempf et al. (Kempf 2007, Clinical Chemistry 53(2): 284-291).

### The results are summarized in the following table:

**Table: Biomarker levels in right heart failure patients having pulmonary embolism or pulmonary hypertension**

| | NT-proBNP pg/ml | | Sensitive Troponin T pg/ml | | GDF-15 pg/ml | | Endoglin ng/ml | |
|---|---|---|---|---|---|---|---|---|
| | P.H. | P.E. | P.H. | P.E. | P.H | P.E. | P.H | P.E. |
| Median | 690 | 3599 | 5.4 | 77.4 | 1580 | 2215 | 6.95 | 4.69 |

| | | | | | | | | |
|---|---|---|---|---|---|---|---|---|
| P.H. : pulmonary hypertension P.E.: pulmonary embolism | | | | | | | | |

## Claims

1. A method of differentiating between pulmonary embolism and pulmonary hypertension as the cause of right heart failure in a subject comprising:
a) determining the amounts of a natriuretic peptide, a cardiac troponin, GDF-15 and Endoglin in a sample of a subject suffering from right heart failure; and
b) comparing the amounts determined in step a) with reference amounts, whereby its differentiated between pulmonary embolism and pulmonary hypertension as the cause of the right heart failure.

2. The method of claim 1, wherein the reference amounts are (i) for the natriuretic peptide: 2500 pg/ml, (ii) for the cardiac troponin: 50 pg/ml, (iii) for GDF-15: 2000 pg/ml and (iv) for Endoglin: 5 ng/ml.

3. The method of claim 2, wherein amounts of the natriuretic peptide, the cardiac troponin and GDF15 larger than or equal to the reference amounts and an amount of Endoglin less than the reference amount are indicative for a pulmonary embolism as the cause of the said right heart failure.

4. The method of claim 2, wherein amounts of the natriuretic peptide, the cardiac troponin and GDF15 less than the reference amounts and an amount of Endoglin larger than or equal to the reference amount are indicative for a pulmonary hypertension as the cause of the said right heart failure.

5. The method of any one of claims 1 to 4, wherein said natriuretic peptide is NT-proBNP.

6. The method of any one of claims 1 to 5, wherein said cardiac troponin is Troponin T.

7. The method of any one of claims 1 to 6, wherein said subject is a human.

8. A method of determining whether a subject suffering from right heart failure is susceptible to a therapy for pulmonary hypertension comprising the steps of the method of any one of claims 1 to 7 and the further step of identifying a subject susceptible to a therapy for pulmonary hypertension if pulmonary hypertension was determined as the cause of the said right hart failure.

9. The method of claim 8, wherein said therapy for pulmonary hypertension is selected from the group consisting of: vasodilator therapies using oxygen, calcium channel blockers, endothelin receptor blockers, natriuretic peptides or calcitonin gene-related peptides; antiinfammatory therapies using prostacyclic analogues, nitric oxide donors, endothelin receptor antagonists, statins, 5-lipoxygenase inhibitors or monocyte-macrophage chemoattractant protein-1; remodelling therapies using nitric oxide donors or endothelin receptor antagonists; inhalation therapies using oxygen, prostacyclin analogues, nitric oxide donors or ethyl nitrite; anticoagulant therapies; and antiplatelet therapies using prostacyclin analogues, nitic oxide donors, L-arginine, phosphodiesterase inhibitors or prostacyclin synthase.

10. A method of determining whether a subject suffering from right heart failure is susceptible to a therapy for pulmonary embolism comprising the steps of the method of any one of claims 1 to 7 and the further step of identifying a subject susceptible to a therapy for pulmonary embolism if pulmonary embolism was determined as the cause of the said right hart failure.

11. The method of claim 10, wherein said therapy for pulmonary embolism is a drug-based thrombolytic therapy or a surgery-based therapy.

12. A device for differentiating between pulmonary embolism and pulmonary hypertension as the cause of right heart failure in a subject comprising:
a) means for determining the amounts of a natriuretic peptide, a cardiac troponin, GDF-15 and Endoglin in a sample of a subject suffering from right heart failure; and
b) means for comparing the amounts determined in step a) with reference amounts, whereby its differentiated between pulmonary embolism and pulmonary hypertension as the cause of the right heart failure.

13. A kit adapted for carrying out the method of any one of claims 1 to 7 comprising:
a) means for determining the amounts of a natriuretic peptide, a cardiac troponin, GDF-15 and Endoglin in a sample of a subject suffering from right heart failure; and
b) means for comparing the amounts determined in step a) with reference amounts, whereby its differentiated between pulmonary embolism and pulmonary hypertension as the cause of the right heart failure.
